# EUROPEAN PATENT SPECIFICATION

(11) **EP 1 796 658 B1**
(45) Date of publication and mention of the grant of the patent: **30.03.2016**
(21) Application number: 05799591.2
(22) Date of filing: 21.09.2005
(51) Int. Cl.: A61K 31/19, A61K 31/20, A61K 31/045, A61K 31/185, A61K 31/22, A61K 31/047, A61P 25/14, A61P 25/16, A61K 31/23, A61K 31/192, A61K 45/06

(54) **DOPAMINERGIC MIMETICS**
DOPAMINERGE MIMETIKA
MIMETIQUES DOPAMINERGIQUES

(30) Priority: 21.09.2004 US 611302 P
(43) Date of publication of application: 20.06.2007
(73) Proprietor: BTG International Limited, London EC4M 7RD (GB)
(72) Inventor: MARTIN, Keith Frank, West Conshohocken, PA19428-2998 (US); HEAL, David John, Nottingham NG1 1GF (GB)
(74) Representative: BTG plc Intellectual Property Group
(86) International application number: PCT/US2005/033860
(87) International publication number: WO 2006/034361

(56) References cited:
- WO-A-00/15216
- WO-A-01/82928
- WO-A-02/051395
- WO-A-2004/077938
- WO-A2-2006/012490
- US-A- 3 665 075
- US-B1- 6 207 856
- US-B2- 6 380 244
- US-B2- 6 670 378
- TIEU K ET AL: "D-[beta]-Hydroxybutyrate rescues mitochondrial respiration and mitigates features of Parkinson disease" JOURNAL OF CLINICAL INVESTIGATION, NEW YORK, NY, US, vol. 112, no. 6, 2003, pages 892-901, XP008085444 ISSN: 0021-9738
- CLOSE S P ET AL: "Failure of SKF 38393-A to relieve parkinsonian symptoms induced by 1-methyl-4-phenyl-1,2,3,6-tetrahydropyridi ne in the marmoset", BRITISH JOURNAL OF PHARMACOLOGY 1985 GB, vol. 85, no. 2, 1985, pages 320-322, XP055049762, ISSN: 0007-1188
- DIMPFEL W ET AL: "Radioelectroencephalographic comparison of memantine with receptor-specific drugs acting on dopaminergic transmission in freely moving rats.", NEUROPSYCHOBIOLOGY 1987, vol. 18, no. 4, 1987, pages 212-218, XP008159252, ISSN: 0302-282X
- DIMPFEL W: "Preclinical data base of pharmaco-specific rat EEG fingerprints (tele-stereo-EEG)", EUROPEAN JOURNAL OF MEDICAL RESEARCH, HOLZAPFEL PUBLISHERS, DE, vol. 8, no. 5, 30 May 2003 (2003-05-30), pages 199-207, XP009122806, ISSN: 0949-2321
- VANITALLIE T B ET AL: "Treatment of Parkinson disease with diet-induced hyperketonemia: a feasibility study.", NEUROLOGY 22 FEB 2005, vol. 64, no. 4, 22 February 2005 (2005-02-22), pages 728-730, ISSN: 1526-632X
- HENDERSON SAMUEL T ET AL: "Study of the ketogenic agent AC-1202 in mild to moderate Alzheimer's disease: a randomized, double-blind, placebo-controlled, multicenter trial.", NUTRITION & METABOLISM 2009, vol. 6, 2009, page 31, ISSN: 1743-7075

## Description

The present invention relates to a method for providing symptomatic relief in the treatment of Parkinson's Disease (PD). More particularly, the invention relates to the unexpected advantages of elevating plasma and brain concentrations of ketone bodies to provide symptomatic relief in the treatment of PD.

PD was first described in 1817 as "the shaking palsy" by the physician James Parkinson. This disease is a progressive neurodegenerative disorder resulting from the death of cells containing the monoamine neurotransmitter, dopamine, in specific regions of the brain. These areas, the substantia nigra and nigrostriatal neuronal pathways, are responsible for the fine control of movement. This loss of dopaminergic function results in a motor syndrome of bradykinesia (slow movements), dyskinesia (abnormal movements), akinesia (rigidity), resting tremor and postural instability. Frequently, the disease also causes depression, dementia, personality changes and speech deficits. The symptoms of PD gradually become more severe with time leading to almost total motor incapacity.

Current drug therapy replaces the deficit in striatal dopamine function to provide symptomatic relief from the motor deficits in PD. Pharmacological strategies for this endpoint include the administration of L-DOPA (the metabolic precursor of dopamine), potentiating synaptic concentrations of dopamine using selective reuptake inhibitors, preventing dopamine metabolism using monoamine oxidase or catechol-O-methyltransferase inhibitors or directly activating dopamine receptors using selective dopamine agonists.

However, none of these drugs is effective in the long term and their use is associated with serious side-effects because they cause overstimulation of nigrostriatal dopamine receptors which have become 'super sensitive' as a result of the neurodenerative process. These side-effects include abnormal motor movements (twitching, writhing, orofacial stereotypies), the "on-off" syndrome (periods of normal movement control followed by periods of rigidity, bradykinesia and tremor), insomnia and drug-induced psychosis.

In the case of the ketone bodies, it is known that they can be utilized by the brain as alternative metabolic fuels to D-glucose and have neurological and psychiatric benefits. For example, it is known that both acute and chronic neurodegenerative states in mammals, eg. man, can be treated by inducing ketosis. Such ketosis can be provided by restriction of diet, eg by starvation or exclusion of carbohydrate, or by administration of ketogenic materials, such as triglycerides, free fatty acids, alcohols (eg butan-l,3-diol), acetoacetate and (R)-3-hydroxybutyrate and their conjugates with each other and further moieties, e.g. esters and polymers of these. Ketogenic materials thus produce a physiologically acceptable ketosis when administered to a patient.

Further therapeutic indications for the application of ketosis include epilepsy, depression, anxiety, schizo-affective disorder, obsessive-compulsive disorder, panic disorder, social anxiety disorder, generalised anxiety disorder and post-traumatic stress disorder, impaired cognitive function resulting from neurodegeneration, pain, diabetes, dystrophies and mitochondrial disorders. In the case of epilepsy, the ketogenic diet has been applied in treatment of intractable seizures with some success for many years, although the mechanism by which the seizure suppression is achieved remains uncertain.

Veech, US 6207856, describes the use of ketone bodies for the treatment of neurodegeneration, inter alia, as caused by toxic proteins such as those found in Parkinson's disease. Veech teaches that such treatment may also cause renervation due to neurotrophic effect on axon regeneration. Tieu et al J.Clin.Invest Sept 2003 Vol 112, No 6 confirms earlier studies by Hiraide et al EP appln 01 122563.8 that ketone bodies may be used to protect CNS mitochondria, such as would be beneficial in oxidative phosphorylation defect induced aspects of Parkinson's disease.

The present inventors have now surprisingly demonstrated that increase in plasma concentrations of ketone bodies produces unanticipated changes in brain electrical activity similar to those evoked by dopaminergic drugs used to provide symptomatic relief in the treatment of PD and other CNS disorders resulting from dopamine deficiency in the brain. Moreover, this invention provides the additional therapeutic advantage that ketogenesis as a clinical treatment for PD occurs with no stimulant effect indicating that ketogenesis will be devoid of the serious dopaminergic side-effects of abnormal motor movements, the "on-off" syndrome, insomnia or drug-induced psychosis because the animals displayed no signs of behavioural stimulation due to dopamine receptor activation at the predicted therapeutic plasma concentrations of ketone bodies.

Thus, whereas the prior art use of ketogenesis applies to treatment of degeneration, the present invention allows adminstration of ketogenic materials for the purpose of providing dopaminergic effect, and may be provided where the degeneration of the substantia nigra and nigrastriatal pathways has progressed beyond the point at which arrest of neurodegeneration would be expected to be effective, at least in the acute setting.

Analysis of brain field potentials ("Tele-Stereo-EEG") has been proven to be a very sensitive tool for the characterization of drug effects on the central nervous system (Dimpfel et al., 1986). After administration of a centrally active drug, quantitative changes in the brain field potentials can be considered as a characteristic fingerprint of that particular drug. "Fingerprints" of more than 100 compounds have been obtained including 8 established drug categories, e.g. stimulants, sedatives, hallucinogenics, tranquilizers, analgesics, antidepressants, neuroleptics, and narcotics. Different dosages of the same drug cause quantitative changes in electrical power. This methodology can, therefore, also demonstrate possible dose-response relationships. Direct comparison with specific reference drugs, or by discriminant analysis with reference to an extensive fingerprint database, permits the detection of any possible similarities with established drugs. In general, "fingerprints" show prominent differences for drugs prescribed for different indications and are similar for drugs with similar indication (Dimpfel, 2003). Furthermore, the pattern of EEG changes in the rat is a useful tool in predicting possible changes in the EEG power spectrum in humans.

Applying this technique to ketogenesis, particularly that induced by direct administration of a ketogenic material such as (R)-3-hydroxybutyrate sodium salt, the present inventors have been able clearly to show EEG changes consistent with the aforesaid action to be efficacious in the in the symptomatic treatment of PD and other CNS disorders resulting from dopamine deficiency in the brain.

Thus in a first aspect of the present invention, there is provided ketogenic material as defined in the appended claim for use in a method of treating a subject in need of acute therapy for acute treament of symptoms of Parkinson's disease such as one or more of motor syndrome of bradykinesia (slow movements), dyskinesia (abnormal movements), akinesia (rigidity), resting tremor, postural instability and speech deficits comprising administering to said subject a therapeutically effective dose of a ketogenic material. Preferably the dose is sufficient to produce a therapeutically effective ketosis.

By acute treatment is particularly included relief of the symptoms during the period of ketosis, that is with onset of relief within minutes or up to two hours of initiation of the ketosis. This is in contrast to treatment having effect after days or months as is envisaged in the prior art.

The ketosis produced is preferably a state in which levels of one or both of acetoacetate and (R)-3-hydroxybutyrate concentrations in the blood of the subject are raised. Preferably the total concentration of these 'ketone bodies' in the blood is elevated above the normal fed levels to between 0.1 and 30mM, more preferably to between 0.2 and 15mM, and most preferably to between 0.5 and 8mM. For the purpose of maximising levels of such compounds in the CNS it is desirable to saturate the transporter through which (R)-3-hydroxybutyrate crosses the blood brain barrier: this occurring at between 1 and 5mM.

In its broadest interpretation, the ketogenic material may be any of those used in the treatment of refractory epilepsy, such as creams and fats combined with low carbohydrate and possibly high protein, e.g. as set out in US 6,207,856 (Veech). However, in order to avoid undesirable consequences of such diets, preferred materials are selected from (R)-3-hydroxybutyrate, salts, esters and oligomers of these. Also disclosed are conjugates of these with other physiologically acceptable moieties, such as carnitine and other amino acids. Other disclosed materials are metabolic precursors of ketones these such as (R)-1,3-butandiol, triacetin, free fatty acids, triglycerides and saccharide esters.

Particular materials are known from the following references as set out in Table 1 below. Doses and formats are as described in the documents identified in the table. Typically the amount of ketogenic material required can be determined by measuring blood levels directly using a meter such as the Medisense Precision Extra (MedisenseInc, 4A Crosby Drive Bedford, MA 01730); BioScanner 2000 (formerly called the MTM BioScanner 1000) from Polymer Technology Systems Inc. Indianapolis, Indiana. In this manner the amount of ketosis derived from a set dose may be ascertained, and that dose iterated to suit the individual.

Typical dose ranges for example might be in the range 5 to 5000mg/kg body weight, particularly for an (R)-3-hydroxybuytrate containing material such as oligomeric (R)-3-hydroxybuytrate or its esters with, e.g. glycerol or (R)-butan-1,3-diol, more preferably 30 to 2000mg/kg body weight, most preferably 50 to 1000mg/kg body weight per day. Doses are conveniently given with meals when orally administered, conveniently before or at the same time as such meals. Regular blood levels are more readily attained by dosing three or four times a day.

In a second aspect of the present invention, there is provided the use of a ketogenic material for the manufacture of a medicament for the acute treatment of Parkinson's disease. Again, suitable ketogenic materials are as described and as exemplified in Table 1.

**TABLE 1**

| Material | Type | Reference |
|---|---|---|
| Sodium-(R)-3-hydroxy-butyrate | Salt | US 4579955 |
| | | US 4771074 |
| (R)-1,3-butandiol | Metabolic precursor | Gueldry & Bralet (1994) Metabolic Brain Diseases 9(2): 171-181 |
| Acetoacetylbutandiol | Metabolic precursor | US 4997976 |
| | | US 5126373 |
| Dimer and trimer BHB | Metabolic precursor | JP 5009185 |
| | | JP 2885261 |
| Acetoacetyltri-3HB | Metabolic precursor | US 6207856 |
| Mid-chain triglyceride | Metabolic precursor | WO 01/82928 |
| Triolide | Metabolic precursor | WO 00/15216 |
| | | WO 00/04895 |
| (R)-3-hydroxybutyrate triglyceride | Metabolic precursor | US 5420335 |
| | | US 6306828 |
| (R)-3-hydroxybutyrate multimers/saccharides | Metabolic precursor | WO 00/14985 |
| | | WO04077938 |

Particularly the medicament is for acute symtomatic relief of motor syndrome of bradykinesia (slow movements), dyskinesia (abnormal movements), akinesia (rigidity), resting tremor and postural instability. By acute treatment is particularly included the reduction of such symptoms during the period in which the ketone body levels are elevated, as opposed to the more chronic or delayed action treatment proposed by the prior art where neurodegeneration is halted or reversed. Other symptoms of PD to be treated by the present method are personality changes and speech deficits.

A third aspect of the present invention provides a pharmaceutical composition for symptomatic treatment of Parkinson's disease, comprising as active ingredient a ketogenic material as defined in the claims.

The composition preferably includes diluent, excipient and/or carrier materials.

The present invention will now be described by way of the following nonlimiting Examples and Figures. Further embodiments falling into the scope of the claims herein will occur to those skilled in the light of these.

### FIGURES

### FIGURES

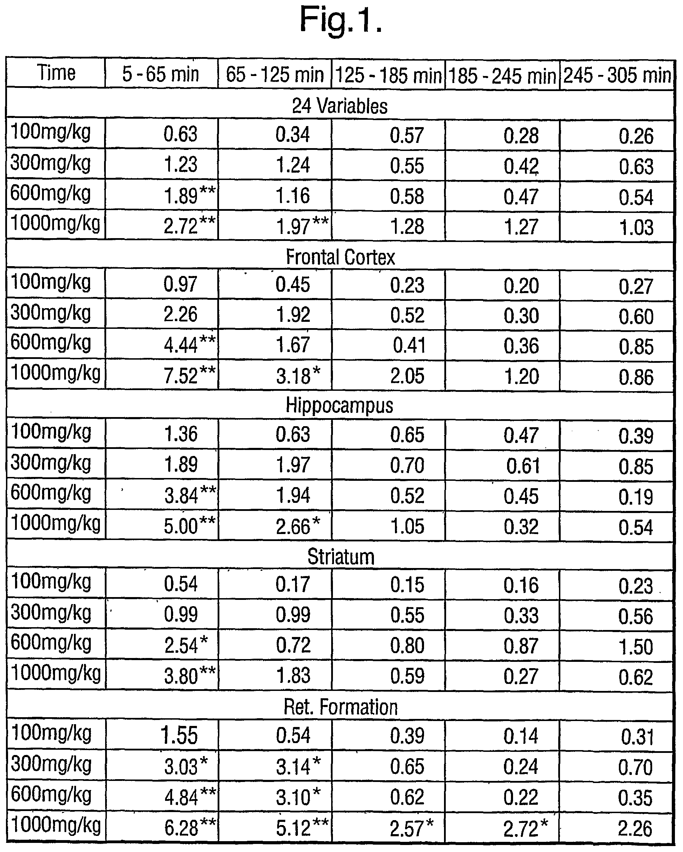

Figure 1: Changes in 24 variables and frequency changes in individual brain regions. Variance/co-variance was estimated on the basis of 88 groups from part of our database of reference drugs with a total of 674 experiments carried out under identical conditions. Variables: frequency range - brain region *F>2.10 corresponds to p<0.05 and **F>2.80 corresponds to p<0.01. For evaluation of 24 variables: *F>1.52 corresponds to p<0.05 and **F>1.79 corresponds to p<0.01. Number of experiments: n=12 (100 mg/kg); n=12 (300 mg/kg); n=11 (600 mg/kg); n=11 (1000 mg/kg). F values - statistics for various time periods after a single intraperitoneal injection of sodium BHB (KTX 0101): 100, 300, 600 or 1000 mg/kg body weight.
Figure 2: Action of vehicle (n=13) on the electrical power of four rat brain areas. Time - dependent changes (percentage change of pre-drug values) in EEG spectral patterns (60 min each) during 300 min after i.p. single-dose application. Definition of frequency ranges: delta (1.25-4.5 Hz, red), theta (4.75-6.75 Hz, orange), alpha1 (7.00-9.50 Hz, yellow), alpha2 (9.75-12.50 Hz, green), beta1 (12.75-18.50 Hz, light blue), beta2 (18.75-35.00 Hz, dark blue).
Figure 3: Action of BHB 100 mg/kg (n=12) on the electrical power of four rat brain areas. Time - dependent changes (percentage change of pre-drug values) in EEG spectral patterns (60 min each) during 300 min after i.p. single-dose application. Definition of frequency ranges see Fig. 2.
Figure 4: Action of BHB 300 mg/kg (n=12) on the electrical power of four rat brain areas. Time - dependent changes (percentage change of pre-drug values) in EEG spectral patterns (60 min each) during 300 min after i.p. single-dose application. Definition of frequency ranges see Fig. 2.
Figure 5: Action of BHB 600 mg/kg (n=11) on the electrical power of four rat brain areas. Time - dependent changes (percentage change of pre-drug values) in EEG spectral patterns (60 min each) during 300 min after i.p.. single-dose application. Definition of frequency ranges see Fig. 2.
Figure 6: Action of BHB 1000 mg/kg (n=11) on the electrical power of four rat brain areas. Time - dependent changes (percentage change of pre-drug values) in EEG spectral patterns (60 min each) during 300 min after i.p.. single-dose application. Definition of frequency ranges see Fig. 2.
Figure 7: Similarity of the "qEEG-fingerprints" of KTX 0101 (sodium BHB) in comparison to different drug classes using discriminant analysis during the period "20th to 50th min after single-dose application". Note the different shading for the classification of different drug actions.
Figure 8: Effect of intraperitoneal injection of various doses of KTX 0101 on plasma concentrations of (R)-3-hydroxybutyrate (BHB). Time-course of action for groups of 4-6 rats. Significantly different from baseline control values by t-test, *p<0.05, **p<0.01, *** p<0.001.
Figure 9: Effect of intraperitoneal injection of various doses of KTX 0101 on plasma concentrations of acetoacetate. Increase 30 minutes after dosing for groups of 6 rats. Significantly different from baseline control values by t-test, *p<0.05, *** p<0.001.

### EXAMPLES

### EEG measurements

Adult Fisher rats (4-6 month of age and day - night converted, bodyweight approximately 400 g) were implanted with 4 bipolar concentric steel electrodes using a stereotaxic surgical procedure. According to the coordinates of Paxinos and Watson (1982), all four electrodes were placed 3 mm lateral within the left hemisphere. Anterior coordinates were 12.2, 5.7, 9.7 and 3.7 mm for frontal cortex, hippocampus, striatum and reticular formation, respectively. A baseplate carrying the electrodes and a 5-pin-plug was fixed to the skull by dental cement attached to 3 steel screws fixed into the skull. Animals were given two weeks for recovery from the surgical procedure.

EEG signals were recorded from frontal cortex, hippocampus, striatum and reticular formation and were amplified and processed as described by Dimpfel et al. (1986). After automatic artefact rejection, signals were collected in sweeps of 4 s duration and submitted to Fast Fourier transformation. The resulting electrical power spectra were divided into 6 frequency ranges: delta (0.8 - 4.5 Hz); theta (4.75 - 6.75 Hz); alpha1 (7.00 - 9.50 Hz); alpha2 (9.75 - 12.50 Hz); beta (12.75 - 18.50 Hz); beta2 (18.75 - 35.00 Hz). Spectra were averaged in steps of 3 minutes each and displayed on-line. In an off-line procedure spectra were averaged to give 15 minute or longer periods for further statistical analysis.

Four doses of KTX 0101 (sodium (R)-3-hydroxybuytrate: 100, 300, 600 and 1000 mg/kg body weight) (supplied by Solvias AG, CH 4002 Basel, Switzerland, batch No: SO-1058.047.1.120) and a vehicle control (0.9% w/v saline) were administered intraperitoneally to a group of 12 animals using a crossover design with at least 3 drug holidays in between the applications. After a pre-drug period of 45 minutes for baseline recording, drug effects were observed continuously for 300 minutes. Changes of electrical power (µV2/W) are expressed as percentage of the 45 minute pre-drug values. Multivariate statistics were calculated according to Ahrens and Läuter (1974).

### Plasma determination of (R)-3-hydroxybuytrate and acetoacetate

One hundred and seventy-one male Sprague-Dawley rats (weight range 200-250g) housed on a standard hour light/dark cycle were used. Animals had free access to a standard pelleted rat diet and tap water at all times. KTX 0101 (sodium (R)-3-hydroxybuytrate; 100, 300, 600 and 1000 mg/kg body weight) supplied by Sigma (H6501, Lot 111K2618) was administered by intraperitoneal injection. Control animals received the appropriate 0.9% saline vehicle via the same route. Animals were killed by CO2 asphyxiation 0h, 0.5h, 1.0h or 2.0h after dosing and a terminal blood sample was collected by cardiac puncture. Blood was taken in lithium heparinised tubes and kept on ice prior to centrifugation to yield the plasma samples for analysis.

Commercial clinical assay kits for the determination of D-β-hydroxybutyrate were obtained from Randox Laboratories (Antrim, UK). The kit quantified NADH via the activity of β-hydroxybutyrate dehydrogenase measured as an increase in OD340nm. An alkaline pH is necessary to drive the reaction equilibrium towards the production of NADH and acetoacetate.

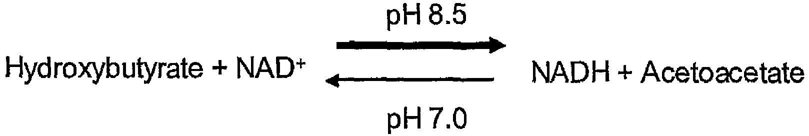

This spectrophotometric assay was modified for application to a 96 well microplate format. The reaction rate was then determined from the increase in OD340nm over a 1 minute time course, after allowing a necessary period for the reaction rate to settle.

The assay developed for the determination of acetoacetate was based on previously published clinical assays (Li et al, 1980; McMurray et al, 1984). A different assay buffer was prepared (0.1M Na2PO4 adjusted to pH 7.0 with HCl) in order to shift the equilibrium of the reaction to production of β hydroxybutyrate and NAD+. Other modifications included the additional use of sodium oxalate at a final assay concentration of 20mM to inhibit lactate dehydrogenase (LDH) present in the plasma samples. The final optimised reagent, therefore, comprised 0.3mM NADH, 20mM oxalate, 0.5 U/ml β hydroxybutyrate dehydrogenase and 0.1M phosphate buffer pH 7.0.

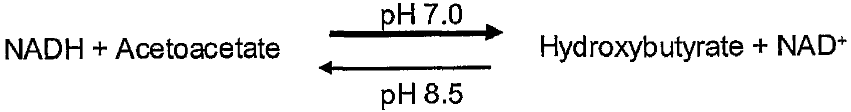

Acetoacetate was measured via the reduction in OD340nm over a 1 minute period after allowing for the reaction rate to settle.

### Results

### EEG measurements

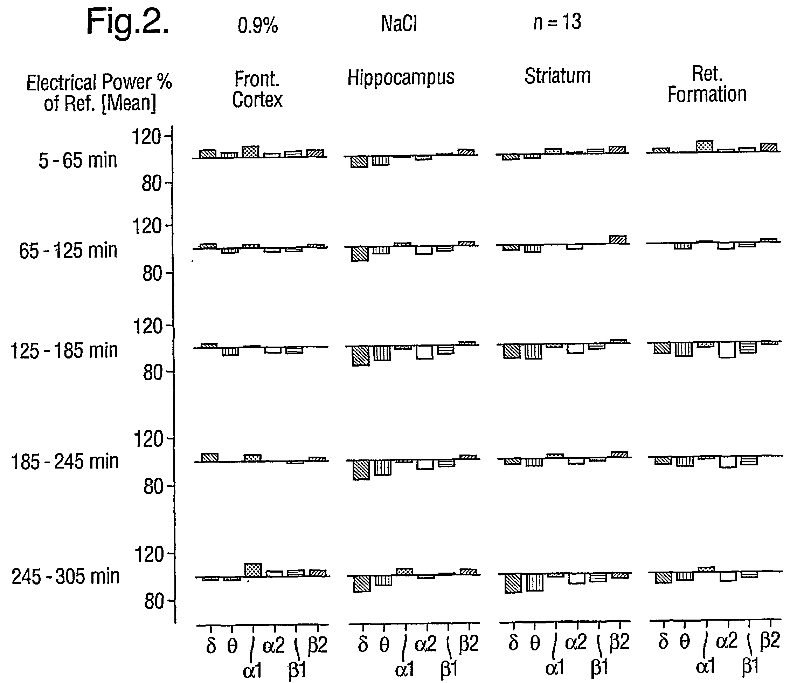

Intraperitoneal administration of 0.9% w/v saline produced no significant changes in the EEG power spectrum in comparison to the predrug values (Fig 2).

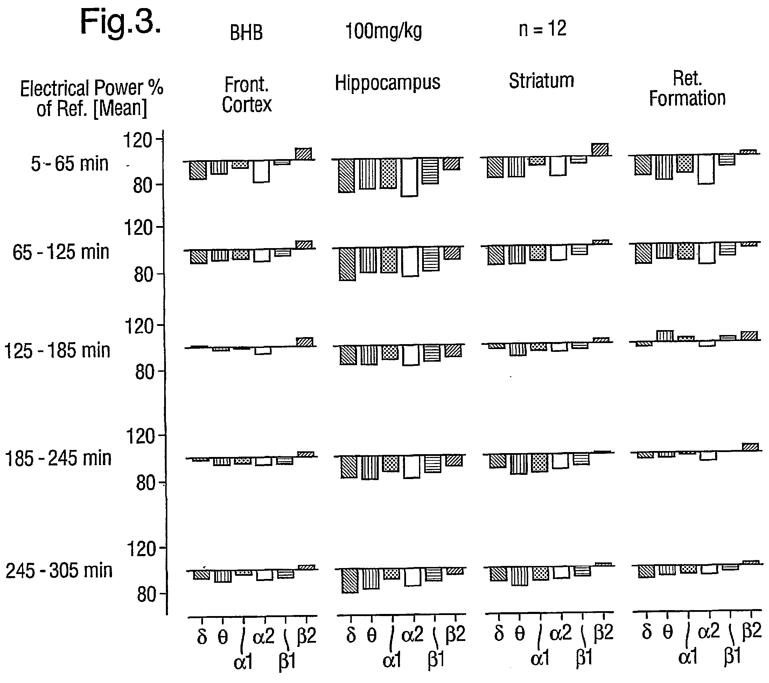

KTX 0101 (100 mg/kg body weight). Administration of this higher dosage of KTX 0101 resulted in frequency changes, especially within the hippocampus and somewhat less within the reticular formation. All regions showed a decrease of electrical power mainly with regard to alpha2 and to a lesser extent with regard to delta frequencies. In the hippocampus theta, alphal and beta1 power also decreased (Fig. 3). The effects lasted for 1-2 hours only. However, these changes were not statistically significant (Fig. 1).

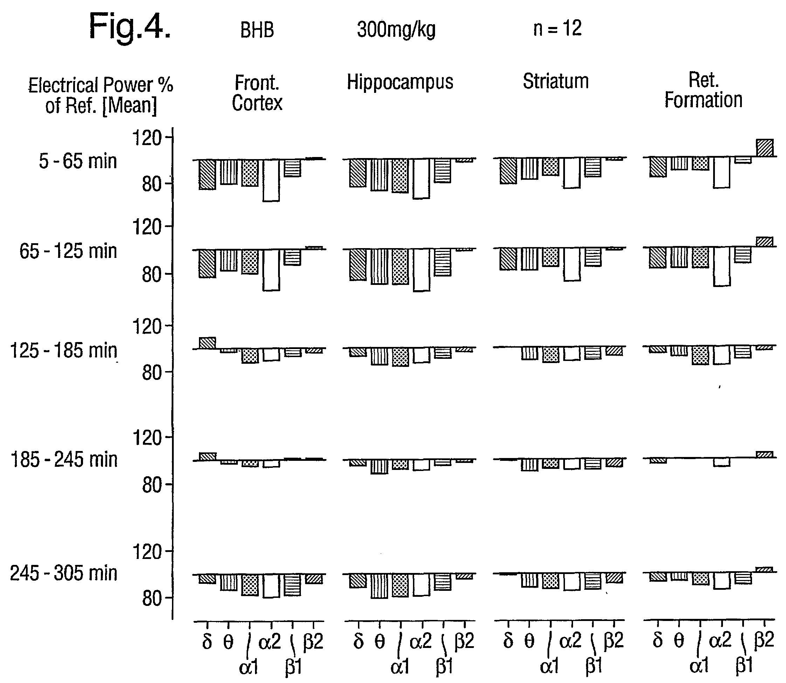

KTX 0101 (300 mg/kg body weight). KTX 0101 300 mg/kg ip produced a consistent pattern of frequency changes characterized by decreases in alpha2 power throughout all brain regions. In addition, delta power changed throughout all regions albeit to a lesser degree. The pattern of changes (Fig. 4) lasted for exactly 2 hours. The changes were only statistically significant in the reticular formation (Fig. 1).

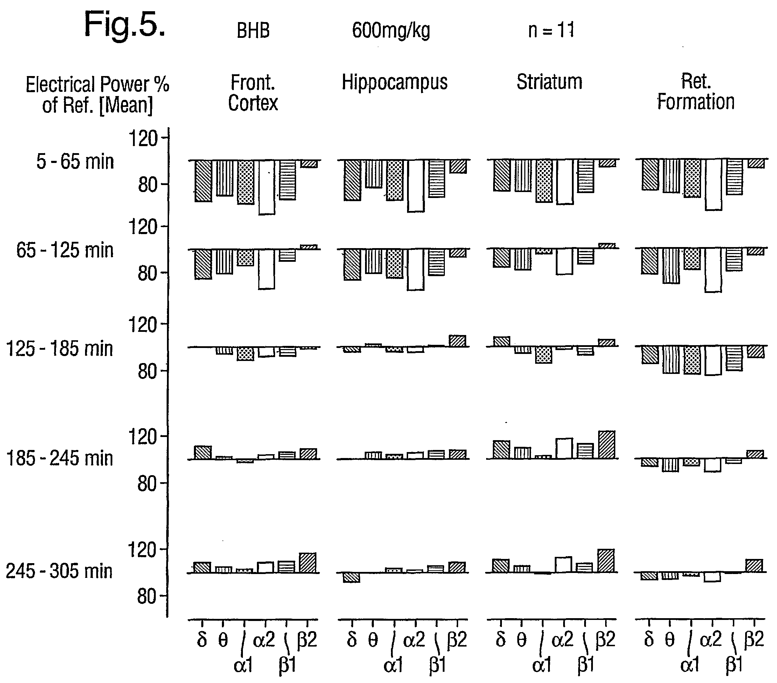

KTX 0101 (600 mg/kg body weight). KTX 0101 600 mg/kg ip produced a similar pattern of change to that seen after 300 mg/kg. The effects generally lasted for 2 hours except for the reticular formation, where decreases in power persisted throughout the third hour (Fig. 5). The results were statistically significant, including the first hour within the striatum. Considering all 24 variables (6 frequencies at all four brain areas), the overall effect was also statistically significant (Fig. 1).

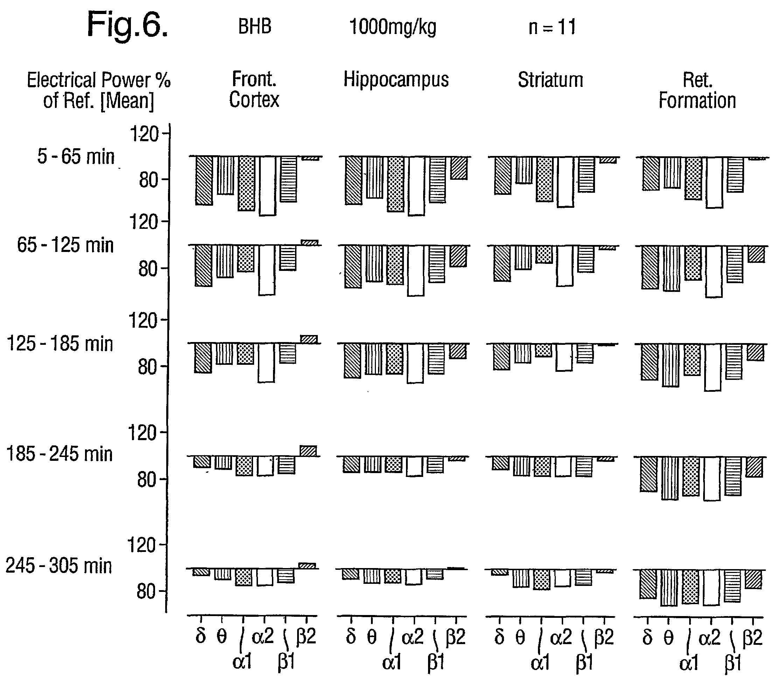

KTX 0101 (1000 mg/kg body weight). Administration of KTX 0101 1000 mg/kg induced an identical pattern of change, but with more prominent decreases of power lasting into the third hour and, with respect to the reticular formation, throughout the total experimental time of 5 hours (Fig. 6). Again, these changes were statistically significant, even for the 4th hour within the reticular formation (Fig. 1).

In summary, clear, dose- and time-dependent statistically significant changes could be observed after the administration of KTX 0101 within a dose range of 300 to 1000 mg/kg ip.

### Plasma concentrations of (R)-3-hydroxybuytrate and acetoacetate

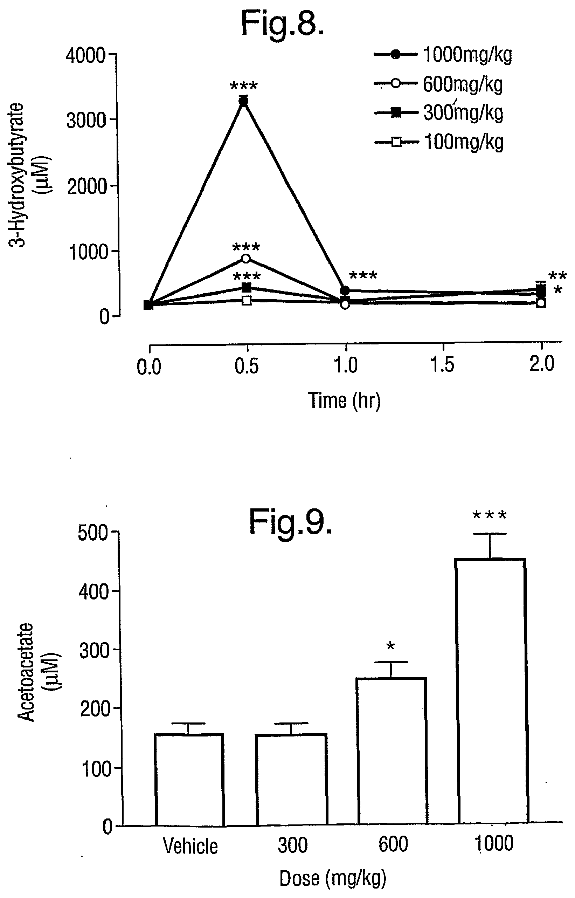

KTX 0101 (100, 300, 600 and 1000 mg/kg ip), when injected via the intraperitoneal route, produced clear dose-dependent increases in the plasma concentration of (R)-3-hydroxybutyrate (Fig. 8). The effect occurred rapidly after injection of KTX 0101 with the highest elevations in (R)-3-hydroxybutyrate being observed in the first 30 min sample. Thereafter, the concentration of this 2 ketone body decreased rapidly and had returned to control values by 1 hour after injection of KTX 0101 (Fig. 8). When the plasma acetoacetate levels were analysed in samples from a subgroup (24) of these rats, KTX 0101 also significantly increased the plasma concentration of acetoacetate 30 min after dosing at doses of 600 and 1000 mg/kg (Fig. 9).

### Discussion

A single intraperitoneal injection of KTX 0101 in the range 100 to 1000 mg/kg induced clear, dose-dependent changes in the EEG power spectrum in freely-moving rats. At the 300 mg/kg dose, these changes were only statistically significant in comparison to vehicle in the reticular formation (Figure 1). However at the 2 highest doses, significant changes were also observed in the frontal cortex, hippocampus and striatum (Figure 1). The changes were maximal in the first 1 hour period after injection of KTX 0101. The observed changes affected all frequencies, except for the beta2 range, with the most prominent effects on the delta and alpha2 frequencies.

With regard to the specific frequency changes observed, drugs acting to enhance dopaminergic function in the brain, eg dopamine precursors (L-DOPA), dopamine releasing agents (amphetamine) or dopaminergic agonists (SKF 38393), all decrease delta, theta and alpha2 frequencies (Dimpfel et al., 1987; Dimpfel, 2003). Thus, decreases in delta, theta and alpha2 activity are also generally associated with an increased behavioural activation and arousal. The ability to enhance dopaminergic function in the brain is the therapeutic mechanism of the major drugs used to provide symptomatic relief in PD, ie L-DOPA, selective dopamine reuptake inhibitors, monoamine oxidase or catechol-O-methyltransferase inhibitors or selective dopamine agonists. The ability of KTX 0101 to decrease the delta, theta and alpha2 EEG frequencies is consistent with the hypothesis that this compound indirectly enhances dopaminergic function in the brain, and as a result of this action, it will be beneficial in the symptomatic treatment of PD and other CNS disorders resulting from dopamine deficiency in the brain.

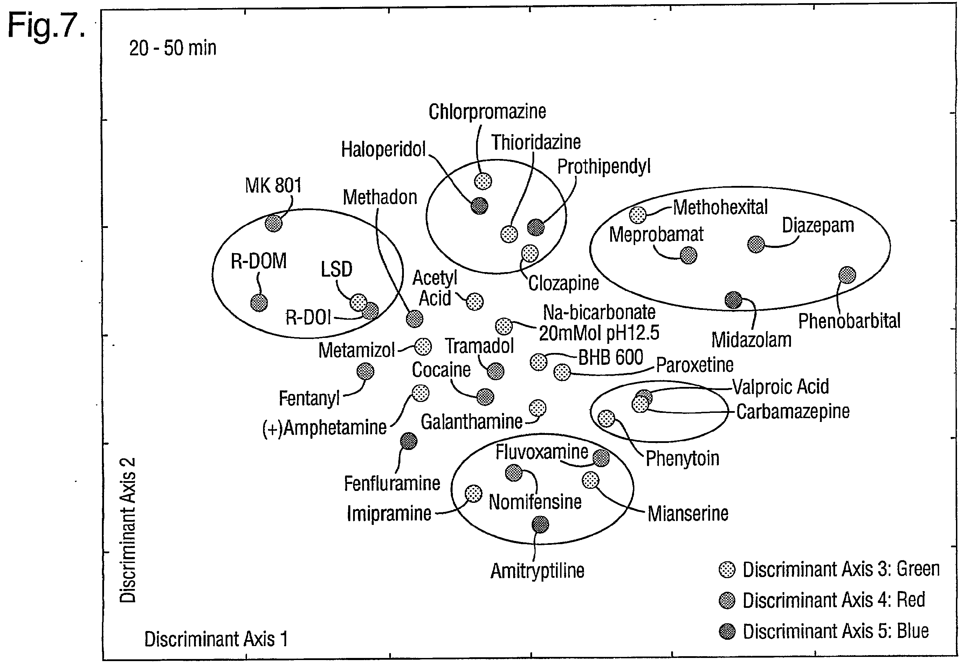

The statistical differentiation of drug action is also possible using the mathematical tool of discriminant analysis. Having 6 frequency ranges and 4 different brain areas the calculations are performed with 24 variables. The results for one time period are shown in Fig. 7. Note that in addition to the 2 projection axes, results from the third to fifth discriminant function are depicted by using an additive colour mixture (similar to that used in colour TV). Thus, not only is a two dimensional projection is used for classification of the EEG "fingerprint", but also the colour. Analysis of the EEG effects of BHB also places it in close proximity to the dopamine reuptake inhibitor, cocaine and the dopamine releasing agent, d-amphetamine (Figure 7), further supporting the hypothesis that KTX 0101 will be of benefit in the symptomatic treatment of PD and other CNS disorders resulting from dopamine deficiency in the brain.

Clear evidence that elevations in the concentrations of ketone bodies are responsible for the observed effects of KTX 0101 on the EEG patterns is provided by the pharmacokinetic analysis of plasma ketone bodies following injection of KTX 0101 (100, 300, 600 and 1000 mg/kg ip). Significant changes in the EEG patterns (Table 2) were only evoked by doses of KTX 0101, ie 300, 600 and 1000 mg/kg, which produced significant elevations of in the levels of plasma ketone bodies, ie (R)-3-hydroxybutyrate and acetoacetate (Figures. 8 and 9). Moreover, the greatest changes in EEG patterns occurred in the period 5 - 65 min (Figure 1) which is consistent with the peak increases in the circulating concentrations of ketone bodies (Figures 8 and 9).

### References

Ahrens H, Läuter J (1974). Mehrdimensionale Varianzanalyse. Akademie-Verlag, Berlin.
Biederman J, Spencer T (1999). Attention-deficit/hyperactivity disorder (ADHD) as a noradrenergic disorder. Pediatr Psychopharmacol Biol Psychiat 1: 1234 - 1242.
Dimpfel W (2003). Preclinical data base of pharmaco-specific rat EEG fingerprints (Tele-Stereo-EEG). Eur J Med Res 8: 199-207.
Dimpfel W, Schober F (2001). Norepinephrine, EEG theta waves and sedation. Brain Pharmacology 1: 89-97.
Dimpfel W, Spüler M, Nickel B (1986). Radioelectroencephalography (Tele-Stereo-EEG) in the rat as a pharmacological model to differentiate the central action of flupirtine from that of opiates, diazepam and phenobarbital. Neuropsychobiology 16: 163 - 168.
Gueldry S, Bralet J (1994). Effect of 1,3-butanediol on cerebral energy metabolism. Comparison with beta-hydroxybutyrate. Metab Brain Dis 9: 171 - 181.
Li PK, Lee JT, MacGillivray MH, Schaefer PA, Siegel JH (1980). Direct, fixed-time kinetic assays for β-hydroxybutyrate and acetoacetate with a centrifugal analyser or a computer-backed spectrophotometer. Clin Chem 26:1713-1717.
McMurray CH, Blanchflower WJ, Rice DA (1984) 1984;. Automated kinetic method for D-3-hydroxybutyrate in plasma or serum. Clin Chem 30:421-425.
Paxinos G, Watson C (1982). The rat brain in stereotactic coordinates, Academic Press, New York.

## Claims

1. A ketogenic material selected from (R)-3-hydroxybutyrate, oligomeric (R)-3-hydroxybutyrate and esters of (R)-3-hydroxybutyrate with glycerol or (R)-butan-1,3-diol, for use in providing acute symptomatic relief for Parkinsons disease symptoms resulting from dopamine deficiency in the brain by administration at a dose of 50 to 1000mg/kg body weight per day for producing a ketosis such that the total concentration of acetoacetate and (R)-3-hydroxybutyrate in the blood is raised to between 0.5 and 8mM;
**characterised in that**
the administration provides a theraputically effective dose of the ketogenic material for the acute symptomatic relief of one or more of the symptoms of motor syndrome of bradykinesia (slow movements), dyskinesia (abnormal movements), akinesia (rigidity), resting tremor, postural instability and speech deficits;
wherein these symptoms are reduced during this period of this ketosis, with the onset of symptomatic relief within up to two hours of initiation of ketosis.

## Patentansprüche

1. Ketogenes Material, ausgewählt aus (R)-3-Hydroxybutyrat, oligomerem (R)-3-Hydroxybutyrat und Estern von (R)-3-Hydroxybutyrat mit Glycerin oder (R)-Butan-1,3-diol, für eine Verwendung im Rahmen der Bereitstellung einer akuten symptomatischen Linderung der Symptome der Parkinson-Erkrankung, die durch einen Mangel an Dopamin im Gehirn hervorgerufen werden, durch die Verabreichung mit einer Dosis von 50 bis 1000 mg/kg Körpergewicht pro Tag zur Erzeugung einer Ketose dahingehend, dass die Gesamtkonzentration an Acetoacetat und (R)-3-Hydroxybutyrat im Blut auf einen Wert zwischen 0,5 und 8 mM gesteigert wird;
**dadurch gekennzeichnet, dass**
durch die Verabreichung eine therapeutisch wirksame Dosis des ketogenen Materials zur akuten symptomatischen Linderung eines oder mehrerer der folgenden Symptome erreicht wird: motorisches Syndrom der Bradykinese (Bewegungsverlangsamung), Dyskinese (Funktionsstörung des Bewegungsablaufs), Akinese (Steifigkeit), Ruhetremor, Haltungsinstabilität und Sprachdefizite;
wobei diese Symptome während dieses Zeitraums dieser Ketose vermindert auftreten, wobei die symptomatische Linderung innerhalb von zwei Stunden nach Beginn der Ketose einsetzt.

## Revendications

1. Matériau cétogène choisi parmi le (R)-3-hydroxy-butyrate, le (R)-3-hydroxybutyrate oligomère et des esters de (R)-3-hydroxybutyrate avec du glycérol ou du (R)-butane-1,3-diol, pour une utilisation dans la fourniture d'un soulagement symptomatique aigu pour les symptômes de la maladie de Parkinson résultant d'un déficit en dopamine dans le cerveau par l'administration à une dose de 50 à 1 000 mg/kg de poids corporel par jour pour la production d'une cétose de telle façon que la concentration totale d'acétoacétate et de (R)-3-hydroxy-butyrate dans le sang est élevée à une concentration entre 0,5 et 8 mM ;
**caractérisé en ce que**
l'administration fournit une dose thérapeutiquement efficace du matériau cétogène pour le soulagement symptomatique aigu d'un ou de plusieurs des symptômes du syndrome moteur de la bradykinésie (mouvements lents), de la dyskinésie (mouvements anormaux), de l'akinésie (rigidité), des tremblements au repos, de l'instabilité posturale et des problèmes d'élocution ;
dans lequel ces symptômes étant réduits durant cette période de cétose, avec l'installation d'un soulagement symptomatique en maximum deux heures après l'initiation de la cétose.
